# EUROPEAN PATENT SPECIFICATION

(11) **EP 3 743 139 B1**
(45) Date of publication and mention of the grant of the patent: **10.08.2022**
(21) Application number: 19700968.1
(22) Date of filing: 24.01.2019
(51) Int. Cl.: A61M 5/315

(54) **MONITORING DEVICE WITH UNIVERSAL ADAPTER TO DRUG INJECTION PENS**
ÜBERWACHUNGSVORRICHTUNG MIT UNIVERSELLEM ADAPTER FÜR ARZNEIMITTELINJEKTIONSSTIFTE
DISPOSITIF DE SURVEILLANCE AVEC ADAPTATEUR UNIVERSEL POUR STYLOS D'INJECTION DE MÉDICAMENTS

(30) Priority: 24.01.2018 EP 18382039
(43) Date of publication of application: 02.12.2020
(60) Divisional application: 22182347.9
(73) Proprietor: Insulcloud, S.L., 28029 Madrid (ES)
(72) Inventor: RUIZ-VALDEPEÑAS MARTIN DE ALMAGRO, Luis, 28029 Madrid (ES); LÓPEZ SÁNCHEZ PASCUALA, José Luis, 28029 Madrid (ES); SOTOMAYOR VÁZQUEZ, Ángel, 28029 Madrid (ES); ANGULO ARAGÓN, Guillermo, 28029 Madrid (ES); BARRANCO ZAFRA, José María, 28029 Madrid (ES); ARENAS LATORRE, Jesús, 28029 Madrid (ES)
(74) Representative: Hoffmann Eitle
(86) International application number: PCT/EP2019/051741
(87) International publication number: WO 2019/145415

(56) References cited:
- WO-A1-2016/193229
- WO-A1-2016/198516
- WO-A1-2018/013419

## Description

### OBJECT OF THE INVENTION

The present invention generally belongs to the field of medicine, and more particularly to the field of means designed for ensuring an appropriate periodic application of a drug by chronic patients, such as the application of insulin in diabetic patients, in order to increase the adherence of the treatment and to improve the quality of life.

An object of the present invention is a novel monitoring device designed to be coupled to a drug application pen of any type or model in the market, in order to provide the patient with useful information to control what kind of drug is injected, how much drug is injected, how it is injected or when it is injected, thereby increasing the therapeutic adherence of said drug and its compliance by the patient. Further, the device is allows to monitor several parameters related to drug administration and application so that the treatment of the patient can be controlled by the patient himself or by caregivers, family members or doctors.

The device of the present invention comprises universal adapter means which can be coupled to any commercial drug pen among the existent solutions in the market, thus providing its users with great versatility and ease of application.

### BACKGROUND OF THE INVENTION

Diabetes mellitus comprises a number of metabolic disorders causing chronic high glucose concentration in blood, also referred to as hyperglycemia, mainly because of an insufficient secretion of insulin by the pancreas (in diabetes type 1) or high periphery resistance to endogenic insulin (diabetes type 2). Currently there are more than 485 million people with diabetes in the world.

Although known insulin treatments have high efficacy on the diabetic patients, one of the biggest challenges for these treatments is increasing the adherence of the treatment to avoid the severe problems due to an uncontrolled disease. This challenge is also present in many diseases which require chronic treatments for their patients.

Many diabetes patients are treated by means of periodic insulin injections. There exists a plurality of devices specially designed for the injection of insulin, although in connection with the present invention mention must be made to the so-called insulin injection pens. These pens are similar to a writing pen having a needle at one end and a pushbutton at the opposite end for actuating an insulin cartridge housed inside the body of the pen. When the patient pushes the pushbutton, a plunger pushes the cartridge for causing the injection of a predetermined amount of insulin through the needle. Each cartridge stores enough insulin for a number of injections, e.g. 300 units of insulin. When the cartridge is empty, it is discarded and replaced by a new full cartridge, or an insulin pen is replaced by a new, full insulin pen.

Although these devices allow for an easy and fast injection of insulin, the patient may forget when a previous injection was carried out, the amount of insulin injected, whether a specific injection was in fact carried out or not, etc. Consequently patients, their parents or tutors in the case of very young patients, or in case of dependent patients, or even their endocrinologist, can be uncertain about the evolution and data concerning said injections. This poses an important drawback, since the injection of an incorrect amount of insulin may have potentially serious consequences for the patient.

The same problem arises in connection with the injection of other drugs in chronic patients who must carry out injections several times a day. As an example, mention can be made to the injection of growth hormone (in short persons), the GLP1 treatment in type 2 diabetic patients, the injection of vitamins in persons with deficient iron absorption, the injection of heparin for preventing venous thrombosis, etc.

In order to solve these problems, there exist electronic monitoring devices comprising adapter means to drug application pens, such as the ones described in patent applications WO 2012/127046 A2 or WO 2016/193229 A1. or WO 2016/198516 A1 These devices generally comprise a detector unit configured to detect an actuation action on the pushbutton of the drug application pen, mostly through the detection of a force, movement and/or a touch applied to said detector unit as part of the pushing action carried out by the user of the drug pen. Known devices further comprise an electronic unit connected to the detector and configured to store and/or provide information related to the detected actuation actions, and wireless communication means which allow transmitting said information by means of devices such as tablets, smartphones, computers, smart watches, smart bracelets, or any other smart device.

Thus, the above-referred monitoring devices provide users with improved monitoring performance, which allows them remotely checking the information associated to the drug application in real time, communicating drug application routines to third persons such as parents, tutors or doctors, and generating alarm notifications when the doses or periodicities of application changes with respect to their scheduled treatment protocols. However, all of these monitoring devices have limitations in terms of their lack of complete universality of application to any drug application pen. This is mainly due to the wide diversity of commercially available models of drug pens available in the market, which normally have own different designs and actuation mechanisms. This means, in practice, that a given monitoring device can normally work only for a given drug pen model, thus being unsuitable for other drug pen designs.

The present invention is aimed to solve the aforementioned limitations of the prior art, through a novel monitoring device which can be coupled to a wide variety of drug application pen models. Although the embodiments described in this document are mainly related to drug pens for the treatment of diabetic patients, it can be also applied without any limitation to other chronic diseases using injection pens, such as multiple sclerosis, fertility methods, growth hormone, etc.

### SUMMARY OF THE INVENTION

According to a first aspect of the invention, there is provided a monitoring device according to claim 1.

### BRIEF DESCRIPTION OF THE INVENTION

The above drawbacks are addressed by the present invention by means of a device capable of coupling to any model of drug application pen and having sensors for automatically detecting physical and/or chemical parameters related to said drug application pen, to the drug comprised in said drug application pen and/or for communicating the patient information about the current, next and/or previous drug injections. Specific features and advantages of the device of the invention will be apparent from the description included in the present document.

In the present document, the term "*drug*" must be widely interpreted to encompass any substance periodically or repeatedly injected into the body of a patient. Specially, the device of the invention is useful for monitoring the injection of drugs in chronic patients, and more particularly for diabetes patients who must receive insulin injections several times a day. However, the term "drug" not only refers to insulin, but also to substances such as GLP1, growth hormone indicated for short persons, vitamins indicated for persons with deficient iron absorption, heparin indicated for persons prone to venous thrombosis, ovarian stimulation substances indicated for persons under in vitro fecundation treatment, as well as other substances indicated for patients with allergies or multiple sclerosis. However, it turned out that in a preferred embodiment, the device is to be used advantageously with insulin.

The term "*pen*", "*drug pen*" or *"drug application pen"* refers indistinctly to a device designed for the injection of drugs in chronic patients. It is an elongated device having a writing pen-like shape comprising a front end having an injection needle and a rear end having an actuation pushbutton. The pen further has a cap, similar to those of conventional writing pens, with a cavity for coupling the front end of the pen in order to cover the injection needle. Some rechargeable pen models housing a disposable cartridge storing the drug have the actuation pushbutton displace a plunger pushing the drug in the cartridge towards the injection needle. Other pen models are wholly disposable, that is, they are discarded when an embedded inner drug reservoir is empty.

A first aspect of the invention discloses a device for monitoring the application of a drug to a patient using a drug pen, the device mainly comprising a main body equipped with several actuation elements, an injection detection means, and a processing means. These components are now disclosed in detail:

### a) Main body:

The main body of the device comprises a coupling element and a piston element that can be dismountably coupled to the drug pen. The term "*dismountably*" refers to the fact that the body may be coupled and uncoupled from the pen several times without causing any damage to the pen. In principle, the main body of the device may be configured in a number of ways provided it can be coupled and uncoupled to any existing pen in the market. In this context, it is important that the body to be coupled to the pen be universal, that is, it be suitable for any pen in the market. The device according to the present invention is compatible with a large amount of commercially available insulin pens such as e.g. KwikPen, FlexTouch, FlexPen and SoloStar, as well as pens for the injection of other drugs or substances.

### b) Injection detection means:

The detection means are implemented mainly through electronic components housed in a core element of the device, and serves to detect one or more physical and/or chemical parameters related to the drug application pen or to a drug comprised in said drug application pen. This detection occurs automatically, that is, the patient does not need to carry out any additional operation further than the usual steps he/she follows when carrying out a drug injection. To this end, the injection detection means is implemented as an actuation detector configured for detecting an actuation of the pen. An actuation detector comprises e.g. a mechanical button provided at the device or, for instance, a capacitive actuator. The skilled person will be able to recognize also different kinds of actuation detectors, i.e. detectors to detect actuation preferably of the pushbutton of the pen to carry out an injection.

### c) Processing means:

The processing means are also comprised in the core element of the device, and they are preferably configured to store the date and time of the injection when the injection detection means detects that a drug injection is carried out. More preferably, the processing means are configured to store a dataset including at least the date and time of the injection when the injection detection means detects that a drug injection is carried out. In order to do this, the processing means is in communication with the injection detection means; additionally, the processing means may further be configured to inform the patient when the next drug injection must be carried out, preferably via a wireless communication means and an external device. Further, the processing means may request information from the patient by several means such as screens, buttons, via a wireless communication means and an external device, and/or links to an external application. Based on said information, the processing means can warn the patient that an injection must be carried out.

The device preferably comprises a real time clock, i.e. a clock adapted to output the date and time of day, connected to the processing means such that the processing means is adapted to store a dataset including at least the date and time of the injection.

In a preferred embodiment, the device is provided with drug kind detection means adapted to detect the kind of applied drug, preferably of applied insulin. In a further preferred embodiment, the device comprises a dosage detection means adapted to detect the amount of charged drug, preferably insulin. In a further preferred embodiment, the device is provided with orientation and/or movement detection means adapted to detect, for instance, the position of the drug pen upon, before or after the injection, or to detect the performance of other actions such as the priming of the drug pen, or the action to remove or couple the needle of the pen.

In a preferred embodiment of the invention, the monitoring device is adapted for its coupling to a drug pen of the type comprising a pushbutton and a rotational dose selector, wherein said monitoring device comprises:
- a coupling element adapted to accommodate the pushbutton and the selector of the drug pen; and
- a core element adapted for housing electronic components comprising one or more sensors adapted to monitor one or more physical and/or chemical parameters related to the drug application pen or to the drug to be injected.

In the present invention the expression "accommodating", referred to the coupling element and its relationship with the pushbutton and the selector, will be understood as any configuration by which both said pushbutton and selector are arranged inside the coupling element.

The expression "housing", referred to the core element and its relationship with the electronic components, will be understood as any configuration, space or structure by which said core element allows the integration, connection and/or activation of the electronic components, and their physical arrangement at or inside the monitoring device.

Advantageously, the device further comprises:
- a piston element at least partially housed in the coupling element and linearly displaceable therefrom, comprising at least one position of said piston element by which it makes contact with the pushbutton of the drug pen when said pen is coupled to the device;
- an external button connected to the piston element and freely rotatable respect the coupling element, configured such that the pushbutton is actuated by pushing said external button, by means of the displacement of the piston element and its contact with said pushbutton.

The expression "partially housed", referred to the piston element and its relationship with the coupling element, will be understood as any configuration by which at least part of the piston element is arranged inside de coupling element.

Thanks to its coupling system and movable parts comprised in the device (namely the piston element and the external button), the device can be adapted to different types of drug pens merely by providing a plurality of gaskets with different shapes and internal diameters. Indeed, the main differences between different drug pen models in connection with the coupling of this configuration of the device are basically differences in diameter and/or shape of the pushbutton and/or dose selector. By providing a set of gaskets of different shapes and internal diameters, a correct coupling of the device of the invention to any pen model is achieved.

The piston element and/or the coupling element comprise one or more guiding means adapted to guide the lineal displacement of the piston element with respect to the coupling element.

In a preferred embodiment of the invention, the coupling element comprises stop means adapted for limiting the amount of linear movement of the piston element with respect to the coupling element.

In a preferred embodiment of the invention, the sensors of the core element comprise one or more of the following: pressure, light, colour, rotation, acceleration, movement, temperature, magnetic field, sound, vibration, ultraviolet, ultrasound, infrared, any kind of encoder, laser, dynamometer, blood parameter sensors (such as glucose meter, pulsioxymeter, cholesterol, etc.) or battery level sensor.

In a preferred embodiment of the invention, the core element comprises an actuation detector comprising a contact element adapted to make contact with the electronic components upon pressure, such that the actuation of the external button causes an electrical contact to close with said electronic components thereby allowing the device to mechanically detect a push action by the user.

The contact element is integrated into a case element.

In a preferred embodiment of the invention, the piston element comprises a monitoring orifice which provides an access for the detection or sensing means equipped in the core element of the device.. More preferably, said core element comprises a bottom printed circuit board (PCB) element comprising lighting means and/or light/colour sensing means arranged facing the monitoring orifice.

The core element comprises one or more case elements (adapted for accommodating the electronic components of the monitoring device, wherein said housing elements are partially or totally housed inside the piston element.

In a preferred embodiment of the invention, the electronic components comprise at least a PCB top and bottom elements connected through a flexible side PCB element, wherein the top PCB element comprises a switch configured for detecting the pushing actions by the patient and/or interacting with said electronic components, for example by means of a contact element when the user pushes the external button. More preferably, the bottom PCB element comprises an accelerometer, magnetometer and a gyroscope arranged forming, for example, an angle of substantially 90° with respect to the central axis of said monitoring device.

In a preferred embodiment of the invention, the external button of the monitoring device is connected to the coupling element by means of a ball bearing joint. This external button can be equipped with one or more magnet elements, adapted for magnetically interacting with the sensors in order to provide them with a magnetic interaction reference.

In a preferred embodiment of the invention, injection detection means is implemented in the device by a mechanical button provided in the cover portion. Preferably, the button may be configured to cover completely the upper surface of the external button. For example, the button may be in electrical communication with the processing means to open or close a contact depending on whether it is pushed or not, such that the processing means immediately receives the information as to whether a drug injection is carried out. An important advantage in connection with the use of a mechanical or capacitive button as an injection detection means is the power savings in comparison with the use of electronic injection detection elements, since the operation and monitoring of the latter require an electrical power source.

According to the invention, when the device of the invention detects a drug injection by the patient, the processing means stores the date and time of the injection, preferably in a storage means provided at the device. Preferably, when the device of the invention detects a drug injection by the patient, the processing means store a dataset including at least the date and time of the injection.

As will be explained below, the device is preferably adapted to store various further parameters in the dataset in addition to the date and the time of drug application. Parameters are selected from the group of kind of applied drug, preferably kind of applied insulin, temperature of drug upon application, preferably temperature of insulin upon application, applied dose of drug, preferably of insulin, duration of drug application, and orientation of the pen at the time of application. As the skilled person will understand, further parameters that are deemed helpful for assisting drug application and/or monitoring drug application can be included in the dataset. In different embodiments of the invention, the parameters can be stored only in response to specific actions by the patient (for example, a drug injection or a priming action), or can be stored continuously during a given period of time (for example, the temperature of the drug before the injection can be continuously monitored during a period of time, and stored to identify eventual temperature changes which can adversely affect the stability the drug).

Storing of a dataset of these parameters in combination with the time and date allows storing of a history of treatment. The dataset can be readout from the device e.g. using a wireless communication means and an external device such as a smartphone or personal computer. Dedicated software provided at the external device enables subsequent use of the dataset e.g. for building graphs, statistical data, etc. As the skilled person will understand, such treatment history is of particular advantage e.g. for a doctor to appropriately control the patient treatment. Providing the treatment history in this form as electronically recorded dataset is advantageous in that the patient is no longer required for example to manually write drug application data into a corresponding paper notebook. In addition, correctness of the data is ensured, i.e. the data cannot be falsified on purpose or unintentionally. Thus, by providing all of these parameters in combination, a particular advantage can be achieved as each one of these parameters is important to achieve an optimal treatment. In addition, in order to enable the device to be capable of storing all of these parameters, it was necessary to construct a device and to find corresponding sensors for each parameter so that the device housing all of these sensors could still be small enough to be coupled to a standard size drug pen such as an insulin pen and could still be conveniently used by a patient.

The date and time of injection, i.e. drug application, and/or the dataset can be readout from the device via wireless communication using an external device such as a user equipment, i.e. for example a mobile device such as a smartphone, a tablet, a smart bracelet, a biometric patch, a smartwatch, a laptop or a personal computer. To this end, in a preferred embodiment, the device further comprises a wireless communication means configured for communicating with an application installed in an external device. This communication means may be implemented in different ways, such as for example by means of Machine to Machine communication, Bluetooth, WiFi, WiMax, NFC, RFID and others. The external device can be of any type, such as user equipment, i.e. a smartphone or mobile phone, a tablet, a computer, and the like. The external device can alternatively or in addition be a server or a group of servers. In any case, as disclosed in detail below, the most preferred configuration entails the communication with a smartphone through Bluetooth. Thus, preferably, the wireless connection is a Bluetooth connection. Preferably, via the communication means, data and parameters such as a desired time of the day for drug application can be inputted by the user using e.g. a smartphone or tablet.

In a preferred embodiment, the device is adapted to store the date and time, preferably the dataset, on at least one external server. As the skilled person will understand, such external server or multiple external servers can form a so called "data cloud" which is a logical storage space provided for a user on such server or on such group of servers which may be provided by a hosting company or the like. The data cloud can be accessed via a general network such as a local communications network provided at home or in a hospital or via the internet. Preferably, the device is adapted, i.e. comprises the mentioned wireless communication means, to wirelessly access the general network, preferably the internet. Other means for data transmission may include, without limitation: RFID, NFC, Bluetooth, radiofrequency, Cloud, LTE, Lora, Sigfox narrow band, GSM, SigLowPan, Zigbee, wireless LAN, UHF Bands IV and V or VHF Band transmission.

In a preferred embodiment, the device is adapted to store the date and time of the injection, preferably the dataset, on at least one server when, or as soon as, the device is connected to the general network, preferably to the internet. In an alternative or additional embodiment, the dataset can be communicated to the at least one server via a wireless connection (e.g. Machine to Machine communication or a Bluetooth connection) to a user equipment such as a smartphone, biometric patch or a smart bracelet, the user equipment being connected to the at least one server via the internet.

In addition to the above described functionality to store a dataset based on various parameters, the device is preferably adapted to warn or remind the user under certain circumstances based on the parameters. Thus, in a preferred embodiment, the device of the invention further comprises an alarm means to notify the patient of certain events. The alarm means may be an acoustic means, visual means, vibration means, etc. As visual means, for example, a LED may be provided. For example, the device may output an alarm at a certain time of day to remind the user to use the pen to apply a drug. In other words, the patient can e.g. program the device for a number of daily injections and the alarm means will warn him/her at the appropriate time. In further embodiments, the alarm can be configured to warn the patient in case that specific actions are not performed correctly, thereby preventing potential decreases in the compliance of a given drug injection protocol. Such actions can be, for instance, a priming or injection action which is not performed at the correct inclination of the drug pen, or an injection action which is not carried out during the expected period of time corresponding to the correct drug dose to be injected with the pen. Also, the alarm means can warn the user in case that a specific dose has been wrongly selected or when the needle is removed or coupled to the insulin pen. The programming of alarms can be accomplished via input of suitable data by the user through an external device such as a smartphone or tablet wirelessly connected to the device. Said external device can also be configured for displaying different suitable alarm means in the form of vibration, push or SMS notifications, telephone call, etc.

In a preferred embodiment, based on the stored date and time of the injection, the alarm means is adapted to output an alarm if a further injection is detected in a predefined time period following the stored date and time of the injection. The predefined time period may be a period of several hours, a day or any other suitable time period between necessary drug injections. Accordingly, a patient is warned immediately if he or she attempts to inject a drug too early or too late such that an unnecessary injection can be avoided or at least the patient can be made aware of the fact that the second injection was too early. Thus, if the user has injected insulin and again wants to inject the same insulin, the device may e.g. output a continuous beep and an LED alarm light will turn red. Thus, the user is warned and informed that the user should not inject insulin.

Further, based on the detection result of the drug kind detection means, the alarm means are preferably adapted to warn a user if the user attempts to inject a wrong kind of drug, preferably insulin, for instance fast acting insulin instead basal insulin.

In a preferred embodiment, the alarm means is adapted to cooperate with the wireless communication means to transfer an alarm to the external device preferably via the at least one external server. For example, in the above case of warning the user of a second, unnecessary injection, a message can be sent to a mobile device and the mobile device can e.g. display to the user: "You've already put this type of insulin. Are you sure you want to inject the insulin again?". Further, in the above example of informing the user of a wrong kind of insulin, a message may be sent to the mobile device which in turn may display "Insulin that you need is "X" and you're trying to put insulin "Y" - are you sure you want to inject this insulin?" In such cases that alarms are output to a user, the user may cancel the alarm e.g. by pushing a corresponding button on the device and may continue with the injection.

For example, the alarm means and the wireless communication means can be adapted such that an alarm message is sent to a remote person, i.e. a person different from a patient using the device, such as a doctor, a caregiver or a family member via Machine to Machine communication, cloud communication, or Bluetooth communication when the device has not been used for a preset time period after a preset date and time. Alternatively or in addition, the alarm means and the wireless communication means can be adapted such that an alarm is communicated to the at least one server which in turn is adapted to automatically sent a message such as an SMS, push notification, an email or a prerecorded telephone call to said person. To this end, the at least one external server is preferably provided with a corresponding software program.

In combination with the alarm means, the device is preferably provided with a charge state detecting means that is adapted to detect and monitor a charge state or filling state or filling level of a drug cartridge of the drug pen. This detection can be carried out either through direct detection means (for example, optical means) or through indirect means (for example, through calculations by the processing means based on a given initial charge volume and subsequent dosages injected by the patient which are subtracted from said initial charge volume). Based on a detection result of the charge state detecting or processing means, the alarm means is preferably adapted to output an alarm when the charge state is below a predetermined threshold. For example, the threshold may be a minimum charge necessary for the application of a dose necessary for one injection. Thereby, mistakes and omission of drug application can be avoided. Thus, based on a detection result of the charge state detecting means, triggered by the alarm means, a message can be sent to a mobile device causing the mobile device to display "Insulin pen finished. Replace it with a new pen".

The device may also request additional information from the patient e.g. through a screen of an external device, for example the amount of drug injected. For example, in the specific case of insulin, the patient may be requested to indicate the amount of insulin injected and the glucose level at that time. This information, along with the information related with the date and time of each injection, can be stored in the storing means either embedded in the processing means or connected thereto, and it can subsequently be used for building graphs, statistical data, etc.

The wireless communication means, on the one hand, allows for sending from the device of the invention both the dataset obtained automatically in connection with the date and time of each injection to the external device. The dataset is preferably processed into information accessible by the patient or the doctor, is converted into a convenient table, into graphs or the like. This function allows the patient to check all the information more conveniently in the external device. After the data is sent to the external device, the data can be processed by a dedicated application on the external device. Alternatively, or in addition, the dataset is processed via a program installed on the at least one server using the dataset stored on the at least one external server. Information resulting from processing the dataset via the cloud service can be acquired by the external device e.g. via the internet. The external device can then display the information to the user of the device e.g. using a dedicated program or application installed on the external device. The data included in the dataset is processed to be accessible to the user, i.e. output of sensors that may be comprised by the device such as temperature sensors, motion sensors (e.g. accelerometers, magnetometer, gyroscopes, etc.) or the like, is processed into output such as numbers included in tables or graphs that can be displayed by the external device using dedicated software or applications. The dataset thus becomes accessible and understandable to the user.

Storing and/or processing of the data via the at least one external server enables synchronizing the treatment history among various external devices such as multiple smartphones e.g. of a parent and of a doctor. Thus, when the patient is e.g. a child, the data can be accessed via a mobile phone of a parent and at the same time via a mobile device or personal computer of a doctor. Thereby, control of the treatment e.g. of a child can be ensured. Similarly, if the patient is an elderly person, family members and a doctor can access cloud data such that treatment can be ensured by multiple persons.

On the other hand, the communication can also take place in the opposite direction, that is, from the application installed in the external device towards the device of the invention. In this case, the communication allows for carrying out configuration adjustments, programming the date and time of the daily programmed injections, requesting specific data such as graphs or the like, updating the software, requesting communication with a doctor, etc.

In addition to the date (e.g. the day, month and year) and the time (e.g. the time of day), in a preferred embodiment, the dataset includes the temperature of the drug, preferably of the insulin, at the time of injection or application, or before or after said injection, either through discrete or continuous analysis or monitoring operation by the sensors. To this end, the device is provided with means for detecting the drug temperature, i.e. for example a temperature sensor and corresponding electronic which is connected to the processing means which in turn is adapted to store the temperature in the dataset. Storing the drug temperature is advantageous for later evaluation of the treatment. In addition, in a preferred embodiment, the alarm means is adapted to warn the user of the pen in the case that at the time of application or at any other moment, the detected drug temperature is above or below a preset threshold.

The monitoring of temperature, as well as any other parameter sensed with the device, can be carried out during the application of the drug, but also at any other time or period of the day, either through discrete or continuous monitoring. This can be helpful to detect cases where the pen has been subject to high temperatures before the injection operation, and where these temperatures can negatively affect the therapeutic effectiveness of the drug (for example, insulin).

In a preferred embodiment, the device further comprises a drug, preferably insulin, kind detection means adapted to automatically detect the kind of applied drug, preferably insulin, when a drug injection is detected, the drug kind detection means being connected with the processing means such that the processing means is adapted to store the kind of applied drug into the dataset.

In a preferred embodiment, the drug is insulin and the drug kind detection means are adapted for automatic detection of the applied insulin.

In a preferred embodiment, the device further comprises a dosage detection means that is adapted to detect the amount of charged drug based on the setting of a dosage actuator provided at the pen. As the skilled person will understand, a drug pen such as an insulin pen is provided with a dosage actuator, e.g. a ring provided at the pen, which can be accessed by the user to set the desired dose. The dosage detection means is e.g. adapted to detect the setting of such dosage actuator to determine the dosage applied at the time of injection. The processing means is further adapted to store the amount of dosage applied into the dataset. Providing the dosage amount as one of the parameters included in the dataset enables e.g. later evaluation of the treatment and/or verifying correctness of the treatment.

The operation of the device of the invention will be better understood from the following description of preferred embodiments.

### BRIEF DESCRIPTION OF THE DRAWINGS

Figs. 1a and 1b show an example of a known insulin pen according to the prior art.
Figs. 2a, 2b and 2c show a general view of a device according to the present invention.
Fig. 3 shows an exploded view of a device according to the present invention.
Figs. 4a, 4b and 4c show up, side and bottom views of the electronic components of the monitoring device comprised in its core element, according to a preferred embodiment of the invention.

### Description of the numerical references used in the drawings:

| | |
|---|---|
| (1) | Monitoring device of the invention |
| (100) | Drug pen, drug pen body |
| (101) | Pushbutton of the drug pen |
| (102) | Cap of the drug pen |
| (103) | Injection needle of the drug pen |
| (104) | Dose selector of the drug pen |
| (105) | Dose indication window of the drug pen |
| (2) | Coupling element of the monitoring device |
| (3) | Piston element of the monitoring device |
| (30) | Monitoring orifice of the piston element |
| (301) | Linear movement of the coupling element |
| (4) | Core element of the monitoring device |
| (40) | Actuation detector of the electronic components |
| (400) | Electronic components |
| (401, 402) | Case elements |
| (410) | Top PCB element |
| (411) | Switch |
| (412) | Processing means |
| (413) | Battery charger |
| (420) | Bottom PCB element |
| (421) | Accelerometer |
| (422) | Gyroscope |
| (423) | Lighting means |
| (424) | Light/colour sensor |
| (425) | Magnetometer |
| (426) | Temperature sensor |
| (427) | Buzzer element |
| (430) | Side PCB connecting element |
| (440) | Battery |
| (5) | External button of the monitoring device |
| (501) | Rotation movement of the external button |
| (6) | Gasket |
| (600) | Inner orifice of the gasket |
| (7) | Closing ring of the coupling element |
| (8) | Rails or guiding means of the piston or coupling element |
| (9) | Retention ring, stop means |
| (10) | Fixation ring |
| (11) | Rotation means, ball bearing joint |
| (12) | Contact element of the external button |

### DETAILED DESCRIPTION OF THE INVENTION

A number of preferred embodiments of the present invention are now disclosed with reference to the drawings. The examples described here are specifically directed to the injection of insulin by an insulin pen in diabetic patients. However, as previously mentioned in this document, the invention must not be interpreted to be limited to insulin pens, as it is applicable to pens intended for the injection of any type of drug. Additionally, the present examples are exemplarily directed to disposable pens.

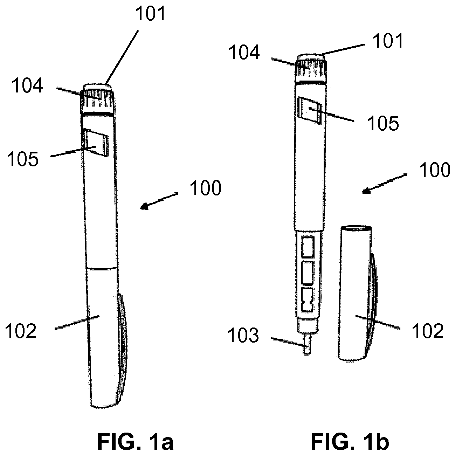

Figs. 1a and 1b show the components of an example of a disposable conventional insulin pen (100) according to the prior art. The pen (100) has an essentially cylindrical main body housing an insulin cartridge. An injection needle (103) is located at a front end of the body of the pen (100) for injecting the insulin stored in the insulin cartridge. In order to do so, the patient pushes a pushbutton (101) located at the rear end of the body of the pen (100) which has normally a unique and characteristic colour for each kind of insulin and insulin pen. The pushbutton (101) actuates in turn an inner plunger connected to a cartridge for injecting the insulin through the needle (103). A cap (102) covers the front end of the pen (100) for preventing accidents with the needle (103).

In order to regulate the insulin dose to be injected, the pen (100) comprises a dose regulation selector (104) which can be rotatably adjusted to set up the insulin dose to be injected. In some embodiments of drug pens (100) (for example in KwikPen, FlexPen or SoloStar commercial models), as the user turns the selector (104), it is moved away from the body of the pen (100) along with the pushbutton (101), at a distance which is proportional to the dose selected. Also, in some commercially available models of drug pen (100) the pushbutton (101) and the dose selector (104) can form a single body (for example, in KwikPen models). In other models (for instance, in FlexTouch devices), the selector (104) has always the same relative position with respect to the main body of the pen (100).

In any of the above-referred drug pen models, when the user pushes the pushbutton (101) the insulin dose will be released and the position of the selector (104) and/or the pushbutton (101) will retract to its original position by undergoing a linear and/or rotating movement.

The rotation of the selector (104) can be indicated through a numerical scale in an indication window (105) comprised in the body of the pen (100). This scale will show the user the number of insulin units that is being charged for injection.

As disclosed below, the monitoring device (1) of the invention is specially designed for automatically detecting when the patient performs an injection action, and can be coupled to any model of insulin pen (100), being thus able to adapt to selectors (104) and pushbuttons (101) of both variable (KwikPen, FlexPen, SoloStar) and fixed (FlexTouch) positions relative to the main body of the pen (100). Additionally, the device of the invention can also adapt to insulin pens (100) where the pushbutton (101) and the dose selector (104) form a single body. This is achieved through coupling means which allow the device (1) fitting to different shapes and/or configurations of said pushbutton (101) and said selector (104) of the insulin pen (100).

Detection means are also provided in the device (1) such that they are activated, for example, by the pressure exerted by the patient when pushing the pushbutton (101) of the insulin pen (100). To this end, said detection means can be implemented as one or more actuation detectors configured for detecting a pushing action on the pushbutton (101) and/or a rotation or linear displacement undergone by the selector (104). The detection means are an essential part of any electronic monitoring device for drug pens, and can adopt several configurations according to different embodiments of the invention. Such means include, for instance: pressure, light, colour, rotation, acceleration, movement, time, temperature or a sensor to detect when the needle is removed or coupled to the insulin pen, among others possibilities. A list of possible embodiments of said detection means and their related electronic, encoding and processing technologies is disclosed, for example, in documents WO 2012/127046 A2 or WO 2016/193229 A1. The sensing and detection technology described in said documents is thus known in the art and will not be discussed in further detail herein.

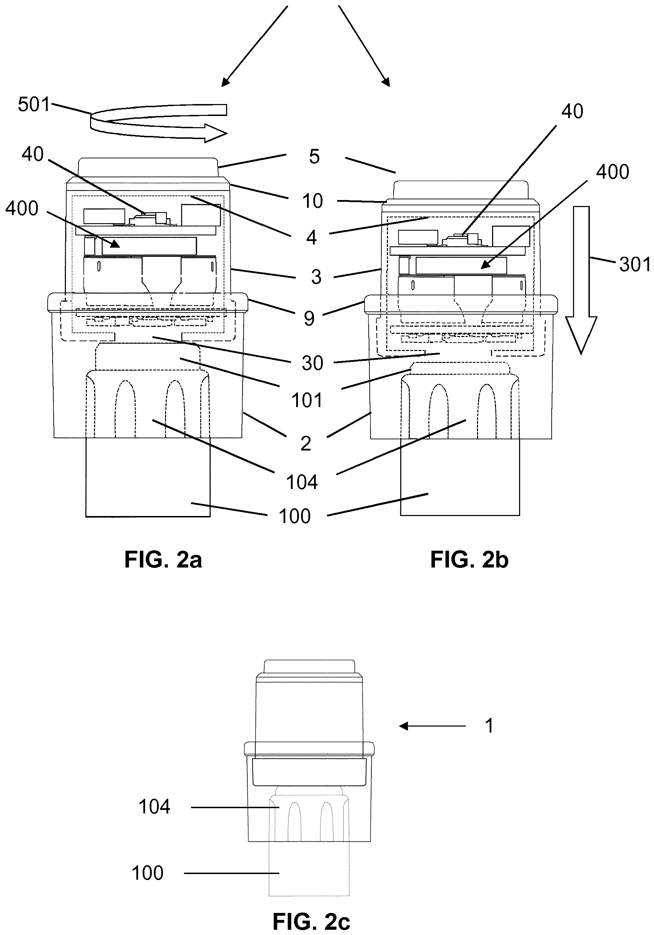

General and exploded views of a preferred embodiment of the monitoring device (1) according to the invention are shown in Figs. 2, 3 and 4. In this embodiment, the device (1) essentially comprises a coupling element (2) adapted for accommodating the pushbutton (101) and the selector (104) of the insulin pen (100); a piston element (3) at least partially housed in the coupling element (2) and linearly displaceable therefrom (see representation of said linear movement by arrow (301) in Fig. 2b, with respect to the rest position shown in Fig. 2a); a core element (4) adapted for accommodating the electronic components (400) within the device (1) (i.e. sensors, processors, encoders, batteries, transmission, communications and charging means, external connections, etc. configured to monitor at least the dose delivered by the drug pen (100)); and an external button (5) connected to the piston element (2) and being freely rotatable respect the coupling element (2) (see representation of said free rotating movement by arrow (501) in Figs. 2a-2b). The combination of linear and rotating movements allowed by respectively the piston element (3) and the external button (5) provides the device (1) with the ability to be applied to drug pens (100) of any configuration, including both variable or fixed dose selectors (104) and pushbuttons (101), as it will be shown below.

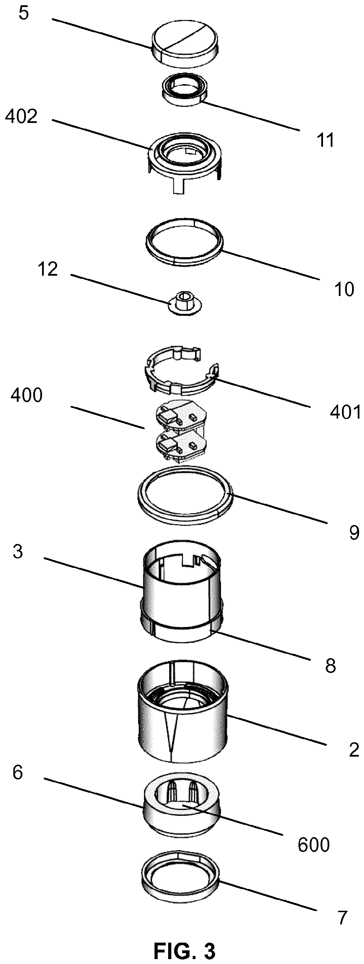

The coupling element (2) has preferably an essentially cylindrical or frustoconical hollow shape, configured for accommodating therein the pushbutton (101) and the dose selector (104) of the drug pen (100). In order to adapt to the specific form of any dose selector (104), the coupling element (2) preferably comprises a gasket (6) (Fig. 3) with an inner orifice (600), whose inner surface is similar to the external surface of the selector (104), such that it can slide along it and embrace it achieving a firm attachment thereto. By employing different designs for the gasket (6), the device (1) of the invention can be used with any available model of drug pen (100). In different embodiments of the invention, the gasket (6) is a removable element which can be detached from the coupling element (for example, to be interchanged with other gaskets (6) for the adaptation of the device for its coupling to different models of drug pens (100)). In order to fix the position of the drug pen (100) inside the coupling element (2), a bottom closing ring (7) can be attached to an end of the coupling element (2) through, for instance, threaded, clipping or clamping means.

As described above, the piston element (3) is housed at least in part inside the coupling element (2) and can be linearly displaced therefrom. This feature is essential for the adaptation of the monitoring device (1) to different designs of drug pens (100), since many of said drug pens (100) comprise selectors (104) and pushbuttons (101) with variable or fixed positions relative to the main body of the pen (100). Thus, they can move away from said main body as the user turns the selector (104), until a distance which is proportional to the dose selected. In order to make contact with any fixed or variable pushbutton (101) configuration, the pushing action of the user will be transmitted from the external button (5) to the piston element (3), which in turn will linearly slide inside the coupling element (2) until making contact with the pushbutton (101) and applying thereto the pressure exerted by the user. Since the device (1) of the invention is coupled to the pushbutton (101) of the pen (100), the pushbutton (101) itself being hidden inside the coupling element (2), when using the pen (100) the user pushes directly the upper portion of the external button (5), and this action can be detected by an actuation detector (40), being pushed by a force that is transmitted by the piston element (3) to the push button of the insulin pen. This way, both elements are actuated at the same time. The actuation detector (40) is thus preferably comprised in the electronic components (400) of the device (1) and can be configured such that a prolonged push is interpreted as an injection for using it as injection detection means. As it can also be seen in Figs. 1a-1b, the piston element (3) preferably comprises a monitoring orifice (30) which provides a monitoring access for the detection or sensing means equipped in the core element (4) of the device (1). The monitoring orifice (30) may comprise a hole, a window to prevent water leaks, or can be made of a transparent material.

In order to guide the linear movement of the piston element (3) inside the coupling element (2), any of them (2, 3) is equipped with guiding means. The guiding means may be one or more cooperating rails (8). This way, the piston element (3) and the core (4) element will rotate jointly with coupling element (2) and therefore, the dosage selector (104). Also, in order to limit the amount of linear movement of the piston element (3), the coupling element (2) can include stop means (9) (for example, a retention ring as shown in Figs. 2 and 3).

The core element (4) accommodates the electronic components (400) of the monitoring device (1) and is preferably comprised into one or more case elements (401, 402) adapted to that purpose. In a preferred embodiment of the invention, the housing elements (401, 402) can in turn be partially or totally housed inside the piston element (3) (as shown in Fig. 3), and fixed to said piston element (3) by means of a fixation ring (10) or any equivalent means thereto. The dotted line of Figs. 2a and 2b referred to the core element (4) should be regarded as a non limiting feature, in the sense that the expression "core element" will be understood as any configuration of electronic components (400) in the monitoring device which comprises one or more sensors configured for monitoring at least the dose delivered by the drug pen (100).

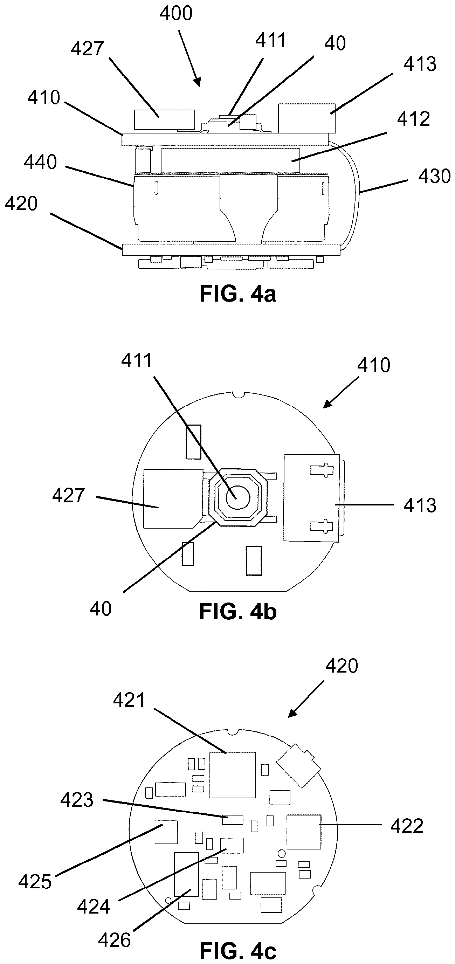

Figs. 4a-4c show top, side and bottom views, respectively, of a possible embodiment of the electronic components (400) of the monitoring device (1) according to the invention. In said figures, the electronic components (400) comprise at least a printed circuit board (PCB) top (410) and bottom (420) elements, optionally connected by a flexible side PCB element (430). Preferably, the top PCB element (410) comprises a switch (411) configured for activating the electronic components (400) by means of a contact element (12) (see Fig. 3) when the user pushes the external button (5). Said contact element (12) can be, for example, a capacitive element. The switch will preferably be located on top of the electronic components (400) to interact with the external button (5). In further embodiments of the invention, the external button (5) can be equipped with one or more magnet elements, adapted for magnetically interacting with the sensors in order to provide them with a magnetic interaction reference. For instance, the magnet element can be a rod arranged at the external button (5) which can interact differently with the sensors depending on the rotational position of the external button (5) when it is pushed by the user. This way, the sensors can detect the position of the user's thumb, of the rotation angle of the external button (5).

In a preferred embodiment of the invention, the core element (4) comprises one or more processing means (412) which may in principle be implemented in a number of ways, such as for example by a microcontroller, a microprocessor, etc. In any case, whether embedded in processing means or as a separate element connected thereto, the monitoring device (1) comprises a control clock for controlling the date and time. Additionally, the device (1) also comprises, either embedded in processing means or as separate elements connected thereto, a communication means, a storing means, and an alarm means.

The storing means of the electronic components (400) allows for storing the information obtained by the device (1), either automatically through the detection of injections, or else manually through the data requested to the patient by means of a touchscreen, an operation program, temporal operation data, etc. In a further example of the invention, a ROM ("Read Only Memory") is used for the operation program, a static memory stores the different elements of the database, alarms, etc., and a RAM ("Random Access Memory") or NVRAM ("Non volatile random access memory") stores temporal operation data such as variables or counters.

The communication means of the electronic components (400) allows for sending the information obtained and stored in the device (1) to an external device, such as for example a smartphone, a tablet or a computer. In this specific example, the communication means can be a Bluetooth chip using a low consumption communication protocol, such as for example protocols 3.0 or 4.0 onwards. On the other hand, the external device has an application, or app, specifically designed for managing the device (1) installed therein. The patient or other person, such as a tutor or a doctor, will be able to view the information stored by the device in a touchscreen installed in the device (1), or by means of the smartphone, tablet or computer. In addition to viewing the information, the patient can change configuration data of the device (1), such as date and time of the alarms, etc. Communication means can be also equipped with direct internet connection configured to send data to the external device. In this way, the monitoring device (1) of the invention can automatically send information in real time without the need of an intermediary device, such as a smartphone, a smart watch, smart bracelet, etc.

The alarm means of the electronic components (400) will warn the patient of an upcoming insulin injection. The alarm means may be implemented in several different ways, comprising for instance a buzzer element (427).

Preferably, a rechargeable battery (440) supplies power to the different electronic components (400) of the monitoring device (1) of the invention. More preferably, the core element (4) of the device is also equipped with a charging or connection port (413) connected to the battery (440) (for example, a USB charging port).

The bottom PCB element (420) preferably comprises one or more of the following sensors and detection means: an accelerometer (421), a gyroscope (422), a lighting means (423) (for example, one or more LED used for illuminating the pushbutton (101) or the dose selector (104), of for signalling/alarm means for the user) and/or a light/colour sensing means (424). Lighting (423) and light/colour sensing means (424) must always be arranged facing the monitoring orifice (30) comprised in the piston (3) element, so that the sensors can receive the information from the pushbutton (101) and/or the selector (104) of the insulin pen (100). Said sensors (421, 422, 424) and detection means (423) can be further combined with a magnetometer (425) and a temperature sensor (426) which are preferably also arranged at the bottom PCB element (420). Also, in a further preferred embodiment of the invention, the accelerometer (421), magnetometer (425) and the gyroscope (422) motion sensors are arranged forming an angle of substantially 90° with respect to the central axis of the monitoring device (1). This configuration favours better calibration and data acquisition for said motion sensors. Thus, light and colour sensors will preferably be located on the bottom part pointing to the pushbutton (101) and as close as possible to each other, while the temperature sensor will be preferably arranged at the bottom part of the electronic components (400) to be as close as possible to the drug pen. The gyroscope must be as close as possible to the central axis of the device (1) to obtain a better signal with less noise, while the accelerometer will preferably be arranged away from the centre of the PCB element (420), to obtain a more significant signal when the dose selector (104) is moved.

By incorporating dedicated sensors as part of the electronic components (400) of the device (1), it becomes possible to ensure compatibility with various pens such as e.g. KwikPen, Flexpen, SoloStar and Flextouch, among others. To achieve this goal, a combination of different sensors can be used, as well as specific software and calibration means in order to monitor the injected dose according to the specific design of the drug pen (100). In this sense, different sensors such as pressure, light, colour, rotation, acceleration, movement, time, magnetic field sensor, temperature, or charge of battery detectors, can be used for this purpose.

The external button (5) of the monitoring device (1) is connected to the piston element (3), and it is freely rotatable with respect to the coupling element (2) by means of rotation means such as, for instance, a ball bearing joint (11). For drug pens (100) comprising a rotatable dose selector (104), the ability of the external button (5) to rotate freely with respect to said selector (104) ensures that its position will remain fixed by the user's finger while he/she is pushing said external button (5). At the same time, the coupling element (2) will rotate integrally with the selector (104) until the dose is fully delivered. In other designs of drug pens (100) where the selector does not rotate when the pushbutton (101) is pushed, the coupling element will also remain stationary.

As a result of the combination of linear and rotating movements allowed by respectively the piston element (3) and the external button (5), the monitoring device (1) of the invention can be easily applied to drug pens (100) of any configuration, including both selectors (104) and/or pushbuttons (101) with both variable and fixed positions.

The external button (5) is implemented as a mechanical actuation detector. The button (5) comprises the contact element (12) adapted to make contact with an electronic board upon pressure (for example, through the switch (411)), such that actuation of the external button (5) causes an electrical contact to close with the electronic components (400) of the core element (4), thereby allowing the device (1) to mechanically detect a push action by the user, optionally triggering further sensors of the device in order to monitor one or more parameters of the associated to the injected dose. In a further embodiment of the invention, a touchscreen can be provided in the uppermost portion of the external button (5).

## Claims

1. Monitoring device (1) adapted for its coupling to a drug pen (100), said pen (100) comprising a pushbutton (101) and a rotational dose selector (104),
wherein the monitoring device (1) comprises at least:
- a coupling element (2) adapted for accommodating the pushbutton (101) and the selector (104) of the drug pen (100), wherein said coupling element (2) comprises an internal gasket (6) with an orifice (600) whose inner surface is adapted to slide along the external surface of the selector (104) and embrace it achieving its attachment thereto;
- a core element (4) adapted for housing electronic components (400) comprising one or more sensors configured for monitoring one or more physical and/or chemical parameters related to the drug pen (100) or to a drug comprised in said drug pen (100);
and **characterised in that** it further comprises:
- a piston element (3) at least partially housed in the coupling element (2) and linearly displaceable therefrom, comprising at least one position of said piston element (3) by which it makes contact with the pushbutton (101) of the drug pen (100) when the device (1) is coupled to said pen (100); wherein the piston element (3) and/or the coupling element (2) comprises one or more guiding means (8) adapted to guide the lineal displacement of the piston element (3) with respect to the coupling element (2); wherein the core element (4) is comprised inside the piston element (3); and wherein the piston element (3) and the core (4) element will rotate jointly with coupling element (2) and therefore, the dosage selector (104); and
- wherein an external button (5) connected to the piston element (3) and freely rotatable respect the coupling element (2), is configured such that the pushbutton (101) is actuated by pushing said external button (5), by means of the displacement of the piston element (1) and its contact with said pushbutton (101), wherein the external button (5) of the monitoring device (1) is connected to the core element (4) by rotation means, and wherein the external button (5) comprises a contact element (12) adapted to make contact with the electronic components (400) upon pressure, such that actuation of the external button (5) causes an electrical contact to close with said electronic components (400) thereby allowing the device (1) to mechanically detect a push action by the user; and wherein the contact element (12) is integrated into a case element (402).

2. Device (1) according to the preceding claim, wherein the internal gasket (6) is interchangeable or removable from the coupling element (2).

3. Device (1) according to the preceding claims, wherein the coupling element (2) comprises a closing ring (7) adapted with threaded, clipping or clamping means for fixing the position of the drug pen (100) inside said coupling element (2).

4. Device (1) according to any of the preceding claims, wherein the sensors of the electronic components (400) comprise one or more of the following: pressure, light, colour, rotation, acceleration, movement, temperature, magnetic field, sound, vibration, ultraviolet, ultrasound, infrared, laser, dynamometer, blood parameter sensor, needle replacement sensor, point of application or battery/drug charge level sensor.

5. Device (1) according to the preceding claim, wherein the piston element (3) comprises a monitoring orifice (30) which provides a monitoring access to the drug pen (100) for the sensors.

6. Device (1) according to the preceding claim, wherein the electronic components (400) comprise a bottom PCB element (420) comprising lighting means (423) and/or light/colour sensing means (424) arranged facing the monitoring orifice (30).

7. Device (1) according to any of the preceding claims, wherein the electronic components (400) comprise at least a top (410) and bottom (420) PCB elements connected through a flexible side PCB element (430), wherein the top PCB element (410) comprises a switch (411) configured for activating said electronic components (400) by means of a contact element (12) as the user pushes the external button (5).

8. Device (1) according to the preceding claim, wherein the bottom PCB element (420) comprises a magnetometer (425), a temperature sensor (426), an accelerometer (421) and a gyroscope (422), wherein the accelerometer (421), magnetometer (425) and the gyroscope (422) are arranged forming an angle of substantially 90° with respect to the central axis of said monitoring device (1).

9. Device (1) according to any of claims 7-8, wherein the top PCB element (410) comprises a rechargeable battery (440) adapted for powering the electronic components (400) and a charging or connection port (413).

10. Device (1) according to any of the preceding claims, wherein the electronic components (400) comprise a wireless communication means configured for communicating with at least one external device, and wherein said at least one external device is a mobile phone, a tablet device, a computer, a smartwatch, a smart bracelet, a biometric patch or an external server.

11. Device according to any of the preceding claims, wherein the external button (5) is equipped with one or more magnet elements adapted to magnetically interact with the sensors.

## Patentansprüche

1. Überwachungsvorrichtung (1), welche für deren Kopplung mit einem Arzneimittelstift (100) angepasst ist, wobei der genannte Stift (100) einen Druckknopf (101) und einen rotierbaren Dosiswähler (104) umfasst,
wobei die Überwachungsvorrichtung (1) mindestens Folgendes umfasst:
- ein Kopplungselement (2), welches für das Unterbringen des Druckknopfs (101) und des Wählers (104) des Arzneimittelstifts (100) angepasst ist, wobei das genannte Kopplungselement (2) eine innere Dichtung (6) mit einer Öffnung (600) umfasst, deren Innenfläche dazu angepasst ist, entlang der Außenfläche des Wählers (104) zu gleiten und sie zu umklammern unter Erreichung deren Befestigung daran;
- ein Kernelement (4), welches für das Aufnehmen von elektronischen Bauteilen (400) angepasst ist, welche einen oder mehrere Sensoren umfassen, welche dazu ausgebildet sind, einen oder mehrere physikalischen und/oder chemischen Parameter bezüglich des Arzneimittelstifts (100) oder eines Arzneimittels, welches im genannten Arzneimittelstift (100) enthalten ist, zu überwachen;
und **dadurch gekennzeichnet, dass** sie zusätzlich Folgendes umfasst:
- ein Kolbenelement (3), welches mindestens teilweise im Kopplungselement (2) aufgenommen ist und davon linear verfahrbar ist, mindestens umfassend eine Stellung des genannten Kolbenelements (3) mittels welcher es einen Kontakt mit dem Druckknopf (101) des Arzneimittelstifts (100) herstellt, wenn die Vorrichtung (1) mit dem genannten Stift (100) gekoppelt ist; wobei das Kolbenelement (3) und/oder das Kopplungselement (2) eins oder mehrere Führungsmittel (8) umfasst, welche dazu angepasst sind, das lineare Verfahren des Kolbenelements (3) in Bezug auf das Kopplungselement (2) zu führen; wobei das Kernelement (4) innerhalb des Kolbenelements (3) enthalten ist; und wobei das Kolbenelement (3) und das Kernelement (4) gemeinsam mit dem Kopplungselement (2) und daher mit dem Dosierungswähler (104) rotieren werden; und
- wobei ein Außenknopf (5), welches mit dem Kolbenelement (3) verbunden ist und frei rotierbar in Bezug auf das Kopplungselement (2) ist, derart ausgebildet ist, dass der Druckknopf (101) betätigt wird indem der genannte Außenknopf (5) gedrückt wird, mittels des Verfahrens des Kolbenelements (1) und dessen Kontakt mit dem genannten Druckknopf (101), wobei der Außenknopf (5) der Überwachungsvorrichtung (1) mit dem Kernelement (4) mittels Rotationsmittel verbunden ist, und wobei der Außenknopf (5) ein Kontaktelement (12) umfasst, welches dazu angepasst ist, einen Kontakt mit den elektronischen Bauteilen (400) unter Druck herzustellen, sodass die Betätigung des Außenknopfs (5) bewirkt, dass sich ein elektrischer Kontakt mit den genannten elektronischen Bauteilen (400) schließt, wodurch es der Vorrichtung (1) erlaubt wird, eine Drückwirkung vonseiten des Benutzers mechanisch zu erfassen; und wobei das Kontaktelement (12) in ein Gehäuseelement (402) integriert ist.

2. Vorrichtung (1) nach dem vorhergehenden Anspruch, wobei die innere Dichtung (6) austauschbar oder vom Kopplungselement (2) abnehmbar ist.

3. Vorrichtung (1) nach den vorhergehenden Ansprüchen, wobei das Kopplungselement (2) einen Abschlussring (7) umfasst, welcher mit Gewinde-, Klammer- oder Klemmmitteln dazu angepasst ist, die Stellung des Arzneimittelstifts (100) innerhalb des genannten Kopplungselements (2) zu fixieren.

4. Vorrichtung (1) nach einem der vorhergehenden Ansprüche, wobei die Sensoren der elektronischen Bauteile (400) einen oder mehrere der folgenden umfassen: Druck-, Licht-, Farb-, Rotations-, Beschleunigungs-, Bewegungs-, Temperatur-, Magnetfeld-, Schall-, Schwingungs-, Ultraviolett-, Ultraschall-, Infrarot-, Laser-, Dynamometer-, Blutparametersensor, Nadelersatzsensor, Applikationsstellen- oder Batterie-/Arzneimittelladezustandssensor.

5. Vorrichtung (1) nach dem vorhergehenden Anspruch, wobei das Kolbenelement (3) eine Überwachungsöffnung (30) umfasst, welche einen Überwachungszugang zum Arzneimittelstift (100) für die Sensoren bereitstellt.

6. Vorrichtung (1) nach dem vorhergehenden Anspruch, wobei die elektronischen Bauteile (400) ein unteres PCB-Element (420) umfassen, welches Beleuchtungsmittel (423) und/oder Licht-/Farbdetektionsmittel (424) umfasst, welche der Überwachungsöffnung (30) zugewandt angeordnet sind.

7. Vorrichtung (1) nach einem der vorhergehenden Ansprüche, wobei die elektronischen Bauteile (400) mindestens ein oberes PCB-Element (410) und ein unteres PCB-Element (420) umfassen, welche über ein flexibles seitliches PCB-Element (430) verbunden sind, wobei das obere PCB-Element (410) einen Schalter (411) umfasst, welcher dazu ausgebildet ist, die genannten elektronischen Bauteilen (400) mittels eines Kontaktelements (12) zu aktivieren, wenn der Benutzer den Außenknopf (5) drückt.

8. Vorrichtung (1) nach dem vorhergehenden Anspruch, wobei das untere PCB-Element (420) ein Magnetometer (425), einen Temperatursensor (426), einen Beschleunigungsmesser (421) und ein Gyroskop (422) umfasst, wobei der Beschleunigungsmesser (421), das Magnetometer (425) und das Gyroskop (422) unter Bildung eines Winkels von im Wesentlichen 90° in Bezug auf die Mittelachse der genannten Überwachungsvorrichtung (1) angeordnet sind.

9. Vorrichtung (1) nach einem der Ansprüche 7-8, wobei das obere PCB-Element (410) eine wiederaufladbare Batterie (440) umfasst, welche dazu angepasst ist, die elektronischen Bauteilen (400) und einen Lade- oder Verbindungsanschluss (413) mit Energie zu versorgen.

10. Vorrichtung (1) nach einem der vorhergehenden Ansprüche, wobei die elektronischen Bauteile (400) ein drahtloses Kommunikationsmittel umfassen, welches dazu ausgebildet ist, mit mindestens einer externen Vorrichtung zu kommunizieren, und wobei die genannte mindestens eine externe Vorrichtung ein Mobiltelefon, eine Tablet-Vorrichtung, ein Computer, eine Smartwatch, ein intelligentes Armband, ein biometrisches Pflaster oder ein externer Server ist.

11. Vorrichtung nach einem der vorhergehenden Ansprüche, wobei der Außenknopf (5) mit einem oder mehreren Magnetelementen ausgerüstet ist, welche dazu angepasst sind, mit den Sensoren magnetisch zusammenzuwirken.

## Revendications

1. Dispositif de surveillance (1) adapté pour son accouplement à un stylo de médicaments (100), ledit stylo (100) comprenant un bouton-poussoir (101) et un sélecteur de dose rotatif (104),
dans lequel le dispositif de surveillance (1) comprend au moins :
- un élément d'accouplement (2) adapté pour contenir le bouton-poussoir (101) et le sélecteur (104) du stylo de médicaments (100), dans lequel ledit élément d'accouplement (2) comprend un joint interne (6) avec un orifice (600) dont la surface interne est adaptée pour glisser le long de la surface externe du sélecteur (104) et le couvrir en réalisant sa fixation à celui-ci ;
- un élément central (4) adapté pour loger des composants électroniques (400) comprenant un ou plusieurs capteurs configurés pour surveiller un ou plusieurs paramètres physiques et/ou chimiques relatifs au stylo de médicaments (100) ou à un médicament compris dans ledit stylo de médicaments (100) ;
et **caractérisé en ce qu'**il comprend en outre :
- un élément de piston (3) au moins partiellement logé dans l'élément d'accouplement (2) et étant linéairement déplaçable à partir de celui-ci, comprenant au moins une position dudit élément de piston (3) par laquelle il fait contact avec le bouton-poussoir (101) du stylo de médicaments (100) lorsque le dispositif (1) est accouplé audit stylo (100) ; dans lequel l'élément de piston (3) et/ou l'élément d'accouplement (2) comprend un ou plusieurs moyens de guidage (8) adaptés pour guider le déplacement linéaire de l'élément de piston (3) par rapport à l'élément d'accouplement (2) ; dans lequel l'élément central (4) est compris à l'intérieur de l'élément de piston (3) ; et dans lequel l'élément de piston (3) et l'élément central (4) tourneront conjointement avec l'élément d'accouplement (2) et, par conséquent, le sélecteur de dosage (104) ; et
- dans lequel un bouton externe (5) connecté à l'élément de piston (3) et librement rotatif par rapport à l'élément d'accouplement (2), est configuré de sorte que le bouton-poussoir (101) est actionné en appuyant sur ledit bouton externe (5), au moyen du déplacement de l'élément de piston (1) et de son contact avec ledit bouton-poussoir (101), dans lequel le bouton externe (5) du dispositif de surveillance (1) est connecté à l'élément central (4) par des moyens de rotation, et dans lequel le bouton externe (5) comprend un élément de contact (12) adapté pour faire contact avec les composants électroniques (400) sur pression, de sorte que l'actionnement du bouton externe (5) cause la fermeture d'un contact électrique avec lesdits composants électroniques (400), permettant ainsi au dispositif (1) de détecter mécaniquement une action de poussée par l'utilisateur; et dans lequel l'élément de contact (12) est intégré dans un élément de boîtier (402).

2. Dispositif (1) selon la revendication précédente, dans lequel le joint interne (6) est interchangeable ou amovible de l'élément d'accouplement (2).

3. Dispositif (1) selon les revendications précédentes, dans lequel l'élément d'accouplement (2) comprend une bague de fermeture (7) adaptée avec des moyens filetés, de clipsage ou de serrage pour fixer la position du stylo de médicaments (100) à l'intérieur dudit élément d'accouplement (2).

4. Dispositif (1) selon l'une quelconque des revendications précédentes, dans lequel les capteurs des composants électroniques (400) comprennent un ou plusieurs des suivants : pression, lumière, couleur, rotation, accélération, mouvement, température, champ magnétique, son, vibration, ultraviolet, ultrason, infrarouge, laser, dynamomètre, capteur de paramètre sanguin, capteur de remplacement d'aiguille, point d'application ou capteur de niveau de charge de batterie/médicament.

5. Dispositif (1) selon la revendication précédente, dans lequel l'élément de piston (3) comprend un orifice de surveillance (30) qui fournit un accès de surveillance au stylo de médicaments (100) pour les capteurs.

6. Dispositif (1) selon la revendication précédente, dans lequel les composants électroniques (400) comprennent un élément de PCB inférieur (420) comprenant des moyens d'éclairage (423) et/ou des moyens de détection de lumière/couleur (424) faisant face à l'orifice de surveillance (30).

7. Dispositif (1) selon l'une quelconque des revendications précédentes, dans lequel les composants électroniques (400) comprennent au moins un élément de PCB supérieur (410) et un inférieur (420) connectés à travers un élément de PCB latéral flexible (430), dans lequel l'élément de PCB supérieur (410) comprend un interrupteur (411) configuré pour activer lesdits composants électroniques (400) au moyen d'un élément de contact (12) lorsque l'utilisateur pousse le bouton externe (5).

8. Dispositif (1) selon la revendication précédente, dans lequel l'élément de PCB inférieur (420) comprend un magnétomètre (425), un capteur de température (426), un accéléromètre (421) et un gyroscope (422), dans lequel l'accéléromètre (421), le magnétomètre (425) et le gyroscope (422) sont disposés en formant un angle de sensiblement 90° par rapport à l'axe central dudit dispositif de surveillance (1).

9. Dispositif (1) selon l'une quelconque des revendications 7-8, dans lequel l'élément de PCB supérieur (410) comprend une batterie rechargeable (440) adaptée pour alimenter les composants électroniques (400) et un port de charge ou de connexion (413).

10. Dispositif (1) selon l'une quelconque des revendications précédentes, dans lequel les composants électroniques (400) comprennent un moyen de communication sans fil configuré pour communiquer avec au moins un dispositif externe, et dans lequel ledit au moins un dispositif externe est un téléphone mobile, un dispositif de type tablette, un ordinateur, une montre connectée, un bracelet intelligent, un patch biométrique ou un serveur externe.

11. Dispositif selon l'une quelconque des revendications précédentes, dans lequel le bouton externe (5) est équipé d'un ou plusieurs éléments magnétiques adaptés pour interagir magnétiquement avec les capteurs.
